(19) Europäisches Patentamt
European Patent Office
Office européen des brevets

(11) EP 4 574 815 A1

(12) EUROPÄISCHE PATENTANMELDUNG

(43) Veröffentlichungstag:
**25.06.2025 Patentblatt 2025/26**

(21) Anmeldenummer: **23219812.7**

(22) Anmeldetag: **22.12.2023**

(51) Internationale Patentklassifikation (IPC):
***C07C 303/44*** *(2006.01)* ***C07C 309/47*** *(2006.01)*

(52) Gemeinsame Patentklassifikation (CPC):
(C-Sets verfügbar)
**C07C 303/44** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Saltigo GmbH**
**51369 Leverkusen (DE)**

(72) Erfinder: **BREDTMANN, Kai Michael**
**42799 Leichlingen (DE)**

(74) Vertreter: **Matzke, Michael**
**LANXESS Deutschland GmbH**
**LEX-IPR**
**Kennedyplatz 1**
**50569 Köln (DE)**

(54) **VERFAHREN ZUR ABTRENNUNG VON AROMATISCHEN SULFONSÄUREN UND DEREN SALZEN AUS WÄSSRIGEN PHASEN**

(57) Gegenstand der Erfindung ist ein einfaches und umweltfreundliches Verfahren zur Abtrennung von aromatischen Sulfonsäuren beziehungsweise deren Salzen aus alkalischen wässrigen Phasen.



Processed by Luminess, 75001 PARIS (FR)

(Forts. nächste Seite)

(52) Gemeinsame Patentklassifikation (CPC): (Forts.)

C-Sets
**C07C 303/44, C07C 309/47**

## Beschreibung

Gegenstand der Erfindung ist ein einfaches und umweltfreundliches Verfahren zur Abtrennung von aromatischen Sulfonsäuren beziehungsweise deren Salzen aus wässrigen Phasen.

Aromatische Sulfonsäuren auf der Basis des Benzol- oder Naphthalin- und Anthrachinon-Gerüsts sind wichtige Zwischenprodukte bei der Herstellung von Farbstoffen, Netz- und Dispergiermittel sowie von Arzneimitten.

Bestimmte aromatische Sulfonsäuren sind auch häufig unerwünschte Nebenprodukte chemischer Reaktionen. Sulfonsäuren entstehen beispielsweise bei der Umsetzung von aromatischen Kohlenwasserstoffen in Gegenwart von Schwefelsäure oder Schwefeltrioxiden, beispielsweise bei Sulfonierungen von Aromaten. So können beispielsweise Tetrahydrochinolin-Derivate säurekatalysiert, beispielsweise in Gegenwart von Schwefelsäure, zu Aminoindan-Derivaten umgelagert werden. Diese Aminoindan-Derivate sind wichtige Zwischenstufen zur Herstellung von Fungiziden, beispielsweise von N-Indanyl-carboxamid-Derivativen. Dabei entstehen jedoch als Nebenkomponenten Sulfonsäure-Derivate der Aminoindane, die wasserlöslich sind und daher bei der Aufarbeitung von Reaktionsgemischen in die Abwässer gelangen.

Teilweise sind diese Substanzen in Wasser löslich, besonders in Form ihrer Salze. Daher entstehen bei chemischen Prozessen, bei denen diese Substanzen eingesetzt werden oder entstehen, oder bei der Anwendung solcher Substanzen häufig Abwässer, die mit diesen Substanzen verunreinigt sind.

Aufgrund der umweltbedenklichen Eigenschaften und der chemischen Stabilität dieser Substanzen kommt in der Regel eine Entsorgung dieser Abwässer in Kläranlagen nicht in Betracht. Als eine technische Lösung zur Entsorgung dieser Abwässer ist deren Verbrennung bei hohen Temperaturen möglich, bei der das Wasser komplett verdampft und die organischen Komponenten thermisch zersetzt werden. Dies erfordert jedoch hohe Mengen Energie.

Zur Abtrennung von aromatischen Sulfonsäuren oder deren Salzen von wässrigen Systemen sind bereits verschiedene Verfahren bekannt. Darunter sind unter anderem Fällungsverfahren zu nennen, bei denen in Wasser unlösliche Salze, beispielsweise Kalziumsalze, erzeugt werden, die dann von dem zu behandelnden Abwasser durch Filtration abgetrennt werden. Diese Verfahren weisen den Nachteil auf, dass dabei in der Regel große Mengen an Feststoffen anfallen, beispielsweise Kalziumcarbonat oder Kalziumsulfat, die aufwändig entsorgt werden müssen. Darüber hinaus sind Extraktionsverfahren, in der Regel mit zwei oder mehreren flüssigen Phasen bekannt. Dabei werden in der Regel wasserunlösliche organische Lösungsmittel verwendet. Oft reicht die Löslichkeit der abzutrennenden Sulfonsäuren in dem organischen Lösungsmittel nicht aus, um diese komplett aus der wässrigen Phase zu entfernen. Daher muss die Löslichkeit der abzutrennenden Substanz in der wässrigen Phase oft durch das sogenannte "Aussalzen" erniedrigt werden. Diese Methode hat jedoch den Nachteil, dass dabei häufig die Phasentrennung unzureichend ist und salzhaltige Abwässer entstehen, die wiederum aufwändig entsorgt werden müssen.

In dem US-Patent US 3,719,703 wird beschrieben, dass man bestimmte Sulfonsäuren im sauren Medium in eine organische Phase extrahieren kann, indem man dieses mit einem höheren tertiären aliphatischen Amin, beispielsweise Tricaprylamin, und einem wasserunlöslichen organischen Lösungsmittel, beispielsweise Chloroform, Ethylacetat, Nitrobenzol, Toluol oder Cyclohexan, behandelt, und die organische Phase, in der sich dann das Amin und die Sulfonsäure oder deren Salz befindet, von der wässrigen Phase abtrennt. Nachfolgend kann die Sulfonsäure aus der organischen Lösung isoliert werden, indem man die organische Phase mit Natronlauge behandelt und die wässrige alkalische Phase zusammen mit dem Natriumsalz der Sulfonsäure abtrennt. Das Verfahren ist für den großtechnischen Maßstab weniger geeignet, vor allem weil dabei große Lösungsmittelmengen anfallen, die ebenfalls mit den Sulfonsäuren verunreinigt sind und ebenfalls aufwändig entsorgt werden müssen.

EP 41134 A2 beschreibt ein Verfahren zur Abtrennung von wasserlöslichen Salzen von aromatischen Sulfonsäuren, beispielsweise m-Nitrobenzolsulfonsäure, 2-Nitro-5-chlor-p-toluolsulfonsäure, $\alpha$-Naphthalinsulfonsäure, aus Sulfiergemischen. Diese Sulfiergemische enthalten neben Wasser und den Produkten Schwefelsäure, die im ersten Schritt mit Wasser auf einen Schwefelsäuregehalt von 2 bis 75 Gew.% im Gemisch verdünnt werden. Anschließend wird die Mischung mit einer äquivalenten Menge eines nicht emulgierend wirkenden, lösungsmittelfreien, wasserunlöslichen Amins mit insgesamt 12 bis 40 Kohlenstoffatomen, das mit der Sulfonsäure ein Salz bildet, versetzt, wodurch eine Phasentrennung verursacht wird. Die Phase, die das Ammoniumsalz der Sulfonsäure enthält, wird abgetrennt, mit wässriger Base im Überschuss versetzt, wodurch die Sulfonsäure wieder aus dem Ammoniumsalz freigesetzt wird und sich von der wässrigen Phase als organische Phase abtrennt, die dann isoliert werden kann. Solche wasserunlöslichen Amine sind beispielsweise Tri-n-octylamin, Tri-2-ethylhexylamin, Tribenzylamin, Methyldioctylamin, Methyldidecylamin, Tridodecylamin, Tributylamin, Di-2-ethylhexylamin, Didecylamin oder N,N-Dibutylanilin. Die meisten primären Amine mit 12 bis 40 Kohlenstoffatomen sind wegen ihrer Emulgierwirkung weniger geeignet. Das Verfahren gemäß EP 41134 A2 weist einerseits den Vorteil auf, dass es lösungsmittelfrei ist. Sind jedoch im Reaktionsgemisch in geringer Menge Neutralsubstanzen, z.B. Diarylsulfonverbindungen, vorhanden, so gehen diese ebenfalls in die organische Phase über. Sie müssen dann mit üblichen Methoden (z.B. Filtration, Destillation) entfernt werden.

In H. Gai et al.: Journal of Cleaner Production 201 (2018) 774-782, wurde die Extraktion von 1-Amino-2-naphthol-4-sulfonsäure aus Abwässern mit Trioctylamin (TOA) mit Methylisobutylketon (MIBK) oder Kerosin untersucht.

Die Effekte des organischen Lösungsmittels, der Konzentration von Trioctylamin, von Temperatur und pH auf den Verteilungskoeffizienten der 1-Amino-2-naphthol-4-sulfonsäure (ANS) wurden untersucht. Dabei stellte sich heraus, dass als organisches Lösungsmittel Methylisobutylketon besser als Kerosin geeignet ist, dass niedrigere Temperaturen besser geeignet für die Extraktion sind als hohe Temperaturen (Optimum: 25 °C), dass eine höhere Menge an Trioctylamin besser ist als eine niedrigere Menge (Optimum: 50% TOA: 50% MIBK), dass ein niedrigerer pH-Wert besser geeignet ist als ein höherer pH-Wert (Optimum: pH 1,1). Unter den optimalen Bedingungen konnte ein Verteilungskoeffizient D von maximal 76 erreicht werden. D ist dabei definiert als der Quotient C(orgANS) / C(aq ANS), wobei C(orgANS) die Konzentration an ANS (in mol/L) in der organischen Phase nach Extraktion und C(aq ANS) in der wässrigen Phase nach Extraktion sind. Ob dieses Verfahren geeignet ist, Spuren an 1-Amino-2-naphthol-4-sulfonsäure in Abwässern aus der wässrigen Phase zu entfernen, ist nicht bekannt. Diese Verfahren benötigt jedoch neben dem Amin TOA noch ein organisches Lösungsmittel (hier MIBK), dass dann wieder energieaufwändig recycliert oder verbrannt werden muss.

Damit stellt sich nach wie vor die Aufgabe, ein verbessertes Verfahren zur Entfernung von aromatischen Sulfonsäuren bereitzustellen, das die Nachteile des Standes der Technik vermeidet.

Überraschenderweise wurde nun ein Verfahren zur Abtrennung von Sulfonsäuren oder deren Salzen aus wässrigen Phasen gefunden, umfassend die Schritte

a) Einstellen des pH-Wertes der wässrigen Phase, in der sich die zumindest eine Sulfonsäure oder deren Salz befindet, auf pH 13 bis 15, und

b) Inkontaktbringen und Vermischen der wässrigen Phase aus Schritt a) mit zumindest einem Amin ausgewählt aus der Gruppe der alicylclischen und aromatischen Amine mit einer Anzahl von Kohlenstoffatomen im Molekül von 5 bis 12 und einer Anzahl von Stickstoffatomen im Molekül von 1 bis 2, wobei sich eine Mischung umfassend zumindest eine wässrige Phase und eine aminische Phase ausbildet, wobei die aminische Phase die zumindest eine abzutrennende Sulfonsäure oder deren Salz enthält, und

c) Trennung der wässrigen Phase und der aminischen Phase.

Das erfindungsgemäße Verfahren richtet sich auf die Abtrennung von Sulfonsäuren oder deren Salzen aus wässrigen Phasen. In diesen wässrigen Phase liegen die Sulfonsäuren je nach pH-Wert der wässrigen Phase in protonierter Form, also in Form der Sulfonsäure, oder bei höheren pH-Werten in Form der Sulfonsäuresalze, auch Sulfonate genannt, vor. Das entsprechende Gegenanion stammt dann aus der wässrigen Phase. In der Regel sind dies Alkalimetallionen, Erdalkalimetallionen oder organische Anionen, beispielsweise Ammoniumionen. Der Nachweis des Gehaltes von Sulfonsäuren oder deren Salzen in den unterschiedlichen Phasen erfolgt in der Regel mittels Flüssigkeitschromatographie, bevorzugt mit HPLC.

Im ersten Schritt des erfindungsgemäßen Verfahrens, also in Schritt a), wird die wässrige Phase auf einen pH-Wert im Bereich von 13 bis 15 gebracht. Die Messung des pH-Wertes erfolgt dabei üblicherweise mit pH-Metern oder Glaselektroden. Da die Messung in überwiegend wässrigem Medium erfolgt, ist die Messgenauigkeit üblicherweise hoch genug. Alternativ kann der pH-Wert einer Lösung auch berechnet werden.

Bevorzugt erfolgt in Schritt a) die Einstellung des pH-Wertes der wässrigen Phase durch Zugabe einer Base ausgewählt aus der Gruppe Alkalimetallhydroxide und Erdalkalimetallhydroxide, bevorzugt aus Natriumhydroxid und Kaliumhydroxid.

Die Base kann bevorzugt in reiner Form oder gelöst in Wasser erfolgen. Da erfahrungsgemäß pH-Wert-Änderungen exotherme Reaktion darstellen, erfolgt die Zugabe der Base bevorzugt unter mechanischem oder hydraulischem Durchmischen der wässrige Phase, Zudosierung der Base zu der gemischten wässrigen Phase und gegebenenfalls durch Kühlung des gesamten Gemisches. Die Base wird dabei üblicherweise in reiner Form zugegeben um das Gesamtvolumen des Gemisches möglichst niedrig zu halten.

Durch den hohen pH-Wert liegt die Sulfonsäure in der wässrigen Phase in Form ihres gelösten und damit dissoziierten Salzes vor, dessen Gegenanion vorwiegend durch das Anion der Base definiert wird.

Ein niedrigerer pH-Wert als pH 13 führt zu einer deutlich schlechteren Phasentrennung des Gemisches nach Zugabe des Amins in Schritt b). Dabei verbleibt ein größerer Anteil des Amins in der wässrigen Phase, was unerwünscht ist. Dagegen sind pH-Werte größer als 15 in wässrigen Lösungen, die Natriumhydroxid als Basen enthalten, rechnerisch nicht möglich. Die Phasentrennung in Schritt c) wird dadurch nicht verbessert. Höhere pH-Werte als 14 lassen sich in der Regel nicht mehr messen, sondern nur noch berechnen. In einer bevorzugten Ausführungsform des erfindungsgemäßen Verfahrens wird in Schritt a) der pH-Wert der wässrigen Phase mit so viel Base, besonders bevorzugt ausgewählt aus der Gruppe Alkalimetallhydroxide, Erdalkalimetallhydroxide, ganz besonders bevorzugt aus Natriumhydroxid, Kaliumhydroxid, erhöht, sodass letztlich nach der Zugabe der Base in der wässrigen Phase, also zu Beginn von Schritt b), ein Überschuss an Hydroxidionen von 1 bis 3 Mol Hydroxidionen pro Liter wässrige Phase, bevorzugt von 1,0 bis 2,0 Mol Hydroxidionen pro Liter wässrige Phase besonders bevorzugt von 1,2 bis 1,8 Mol Hydroxidionen pro Liter wässrige Phase

vorliegt.

Schritt a) erfolgt vorzugsweise bei einer Temperatur von 0 bis 70 °C, bevorzugt von 15 bis 60 °C, besonders bevorzugt von 20 bis 50 °C.

Im nachfolgenden Schritt b) erfolgt das Inkontaktbringen der wässrigen Phase aus Schritt a) mit einem Amin ausgewählt aus der Gruppe der alicylclischen und aromatischen Amine mit einer Anzahl von Kohlenstoffatomen im Molekül von 5 bis 12 und einer Anzahl von Stickstoffatomen im Molekül von 1 bis 2.

Bevorzugt ist das Amin ausgewählt aus der Gruppe Cyclohexylamin, Isophorondiamin und Anilin.

Auch Schritt b) ist exotherm. Daher erfolgt die Zugabe des Amins bevorzugt unter mechanischem oder hydraulischem Durchmischen der wässrige Phase, Zudosierung des Amins zu der gemischten wässrigen Phase aus Schritt a) und gegebenenfalls unter Kühlung des gesamten Gemisches. Das Amin wird dabei üblicherweise in reiner Form zugegeben um das Gesamtvolumen des Gemisches möglichst niedrig zu halten.

Schritt b) erfolgt vorzugsweise bei einer Temperatur von 0 bis 70 °C, bevorzugt von 15 bis 60 °C, besonders bevorzugt von 20 bis 50 °C.

Während der Zugabe und/oder nach beendeter Zugabe des Amins in Schritt b) erfolgt eine Vermischung des daraus resultierenden Gemisches, das im Ruhezustand zwei Phasen ausbildet: eine wässrige Phase und eine aminische Phase. Die "aminische Phase" kann hier erfindungsgemäß gleichbedeutend auch als "organische Phase" bezeichnet werden. Diese Vermischung erfolgt üblicherweise mechanisch, beispielsweise mit einer Rührvorrichtung, oder hydraulisch, beispielsweise durch Umpumpen. Wichtig ist dabei, dass während des Vermischens die Bildung von zwei Phasen, die das Gemisch ohne ausreichende Durchmischung bilden würde, vermieden wird.

Während des Vermischens des zweiphasigen Gemischs aus Schritt b) erfolgt überraschenderweise ein Übergang der Sulfonsäure oder deren Salze aus der wässrigen Phase in die aminische Phase.

Zur quantitativen Beschreibung der extraktiven Abtrennung ist die Extraktionszahl E eine geeignete Größe. Diese wird erfindungsgemäß als Quotient der absoluten Substanzmassen der Sulfonsäure m(org) in der organischen und m(aq) in der wässrigen Phase mit den Volumina V(org) der organischen Phase und V(aq) der wässrigen Phase definiert. Dabei ist c(org) die Konzentration [Mol/Liter] der Sulfonsäure der in der organischen Phase und c(aq) die Konzentration der Sulfonsäure in der wässrigen Phase:

$$E = m(org) / m(aq) = c(org) \times V(org) / c(aq) \times V(aq)$$

Dabei ist die Substanzmasse bzw. die Konzentration der Sulfonsäure in der jeweiligen Phase erfindungsgemäß berechnet als Summe der ionisierten und nicht ionisierten Verbindung, also der Säure in ihrer neutralen Form und in Form ihres Salzes.

In dem erfindungsgemäßen Verfahren werden ungewöhnlich hohe Extraktionszahlen von größer als 100, bevorzugt größer als 500, erreicht. In nicht erfindungsgemäßen Vergleichsversuchen mit anderen Aminen, beispielsweise Triethylamin oder Tributylamin, liegen die Extraktionszahlen dagegen bei maximal 10.

Für das erfindungsgemäße Verfahren ist es unerheblich, ob in der wässrigen Phase, die in Schritt a) eingesetzt wird, weitere anorganische Säuren, beispielsweise Schwefelsäure, enthalten sind oder nicht. Sind in der wässrigen Phase, die in Schritt a) eingesetzt wird, weitere anorganische Säuren enthalten, muss dementsprechend in Schritt a) mehr Base eingesetzt werden, um auch die anorganische Säure zu neutralisieren. Dabei entstehen in der wässrigen Phase Salze, die aber die Extraktion der Sulfonsäure in die organische Phase nicht beeinflussen.

Die Dauer des Vermischens in Schritt b) beträgt beispielsweise von 5 Minuten bis 8 Stunden, bevorzugt von 1 bis 6 Stunden. Durch eine Inprozesskontrolle des Gehalts an Sulfonsäure in wässriger und aminischer Phase kann die optimale Dauer der Vermischung einfach bestimmt werden.

Bevorzugt beträgt das Massenverhältnis zwischen wässriger Phase und aminischer Phase in Schritt b) von 1 : 1 bis 25 : 1, besonders bevorzugt von 3 : 1 bis 15 : 1. Aufgrund der hohen Extraktionszahl der Sulfonsäure zwischen aminischer und wässriger Phase sind niedrigere Massenverhältnisse, also eine höhere Masse an Amin bezogen auf die wässrige Phase nicht erforderlich, sondern machen das Verfahren weniger ökonomisch.

Nach erfolgter Vermischung in Schritt b) des erfindungsgemäßen Verfahrens wird in Schritt c) die sich aus dem Gemisch bildenden wässrigen Phase von der aminischen Phase abgetrennt, wobei die aminische Phase die abzutrennende Sulfonsäure oder deren Salz enthält und die Sulfonsäure in der wässrigen Phase aufgrund der hohen Extraktionszahl der Sulfonsäure zwischen aminischer und wässriger Phase nur noch in geringen Mengen vorhanden ist. Die abgetrennte wässrige Phase kann für die weitere Verwendung in anderen Prozessschritten, beispielsweise Ableitung in eine Kläranlage oder Isolierung von gewünschten Reaktionsprodukten, unbedenklich eingesetzt werden.

Die aminische Phase aus Schritt c) kann bevorzugt ohne weitere Behandlung in einen neuen Schritt b) wieder eingesetzt werden, ohne dass sich die Extraktionszahl wesentlich ändert. Üblicherweise kann eine aminische Phase ohne weitere Behandlung bis zu zwanzig Mal oder öfter in eine neues Trennverfahren eingesetzt werden. Durch Kontrollen der Konzentrationen der Sulfonsäure in beiden Phasen kann der Fachmann anhand der individuellen Gegebenheiten und

Anforderungen die Anzahl der möglichen Wiederholungen leicht ermitteln. Nach dem letzten Einsatz der aminischen Phase in die Schritte b) und c) des erfindungsgemäßen Verfahrens kann diese beispielsweise als Abfall, beispielsweise durch thermische Zersetzung, entsorgt werden.

Das erfindungsgemäße Verfahren kann bevorzugt für folgende Ausführungsformen mit den oben beschriebenen Parametern und Bedingungen angewendet werden.

Beispielsweise ist die Sulfonsäuren eine Verbindung der Formel (I),

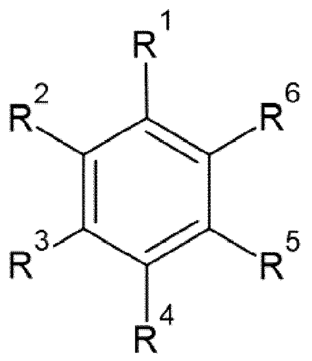

(I),

in der zumindest einer der Reste $R^1$, $R^2$, $R^3$ oder $R^4$ für SOsX steht, und

in der die Reste $R^1$, $R^2$, $R^3$ und $R^4$, die nicht für SOsX stehen, jeweils unabhängig voneinander für Wasserstoff, Amino, Nitro, Fluor, Chlor, Brom oder Alkyl stehen und

in der $R^5$ und $R^6$ zusammen mit den Kohlenstoffatomen, mit denen sie verbunden sind, für einen anellierten alizyklischen 5-gliedrigen bis 7-gliedrigen Ring oder einen 6-gliedrigen aromatischen oder heteroaromatischen Ring bilden, der selbst ein oder mehrfach mit den Resten $R^7$, $R^8$, $R^9$ und $R^{10}$ substituiert sein kann, wobei

$R^7$, $R^8$, $R^9$ und $R^{10}$ jeweils unabhängig voneinander für COOX, SOsX, Hydroxy, Amino, Nitro, Fluor, Chlor, Brom oder Alkyl stehen

und in der X für Wasserstoff, ein Alkalimetallion, ein Erdalkalimetallion oder ein Ammoniumion steht,

Bevorzugt ist die Verbindung der Formel (I) eine Verbindung der Formel (II),

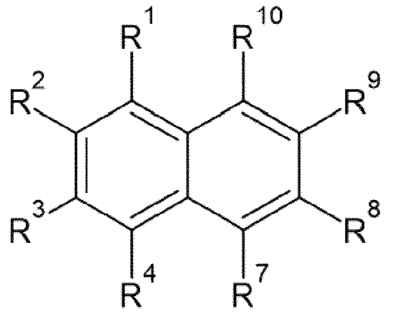

(II),

in der die Reste $R^1$, $R^2$, $R^3$, $R^4$, $R^7$, $R^8$, $R^9$ und $R^{10}$ die für Formel (I) genannte Bedeutung haben.

Besonders bevorzugte Verbindungen der Formel (II) sind 2-Aminonaphthalin-1-sulfonsäure, 4-Amino-3-hydroxynaphthalin-1-sulfonsäure, 4-Amino-5-hydroxynaphthalin-1-sulfonsäure, 4-Aminonaphthalin-1-sulfonsäure, 4-Hydroxynaphthalin-1-sulfonsäure, 5-Aminonaphthalin-1-sulfonsäure, 5-Hydroxynaphthalin-1-sulfonsäure, 6-Aminonaphthalin-1-sulfonsäure, 7-Aminonaphthalin-1-sulfonsäure, 7-Hydroxynaphthalin-1-sulfonsäure, 8-Aminonaphthalin-1-sulfonsäure, 4-Amino-5-hydroxynaphthalin-1,3-disulfonsäure, 6-Aminonaphthalin-1,3-disulfonsäure, 7-Aminonaphthalin-1,3-disulfonsäure, 7-Hydroxynaphthalin-1,3-disulfonsäure, 2-Aminonaphthalin-1,5-disulfonsäure, 3-Aminonaphthalin-1,5-disulfonsäure, 4-Aminonaphthalin-1,5-disulfonsäure, 4-Hydroxynaphthalin-1,5-disulfonsäure, 4-Aminonaphthalin-1,6-disulfonsäure, 4-Hydroxynaphthalin-1,6-disulfonsäure, 8-Aminonaphthalin-1,6-disulfonsäure, 8-Hydroxynaphthalin-1,6-disulfonsäure, 2-Amino-5-hydroxynaphthalin-1,7-disulfonsäure, 4-Amino-5-hydroxynaphthalin-1,7-disulfonsäure, 4-Aminonaphthalin-1,7-disulfonsäure, 1-Hydroxynaphthalin-2-sulfonsäure, 4-Aminonaphthalin-2-sulfonsäure, 5-Aminonaphthalin-2-sulfonsäure, 6-Amino-4-hydroxynaphthalin-2-sulfonsäure, 6-Aminonaphthalin-2-sulfonsäure, 6-Hydroxynaphthalin-2-sulfonsäure, 7-Amino-4-hydroxynaphthalin-2-sulfonsäure, 7-Aminonaphthalin-2-sulfonsäure, 7-Hydroxynaphthalin-2-sulfonsäure, 8-Amino-4-hydroxynaphthalin-2-sulfonsäure, 8-Aminonaphthalin-2-sulfonsäure, 4-Aminonaphthalin-2,6-disulfonsäure, 1-Aminonaphthalin-2,7-disulfonsäure, 3,5-Dihydroxynaphthalin-2,7-disulfonsäure, 3-Amino-5-hydroxynaphthalin-2,7-disulfonsäure, 3-Aminonaphthalin-2,7-disulfonsäure, 3-Hydroxynaphthalin-2,7-disulfonsäure, 4,5-Dihydroxynaphthalin-2,7-disulfonsäure, 4-Amino-5-hydroxynaphthalin-2,7-disulfonsäure, 4-Aminonaphthalin-2,7-disulfonsäure, 4-Hydroxynaphthalin-2,7-disulfonsäure, 8-Aminonaphthalin-1,3,5-trisulfonsäure, 7-Aminonaphthalin-1,3,6-trisulfonsäure oder 8-Aminonaphthalin-1,3,6-trisulfonsäure.

Ebenfalls bevorzugt ist die Verbindung der Formel (I) eine Verbindung der Formel (III),

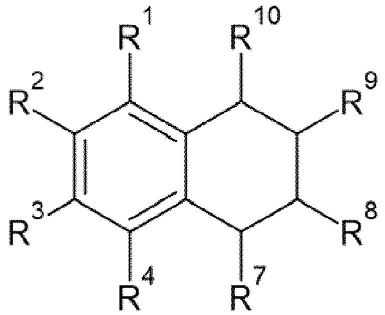

(III),

in der die Reste $R^1$, $R^2$, $R^3$, $R^4$, $R^7$, $R^8$, $R^9$ und $R^{10}$ die für Formel (I) genannte Bedeutung haben.

Ebenfalls bevorzugt ist die Verbindung der Formel (I) eine Verbindung der Formel (IV),

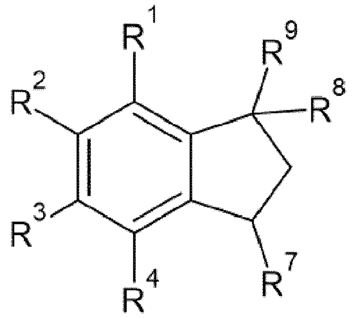

(IV),

in der zumindest einer der Reste $R^1$, $R^2$, $R^3$ oder $R^4$ für SOsX steht, und
in der die Reste $R^1$, $R^2$, $R^3$ und $R^4$, die nicht für SOsX stehen, und die Reste $R^7$, $R^8$ und $R^9$ die für Formel (I) genannte Bedeutung haben.

Besonders bevorzugt ist die Sulfonsäure der Formel (I) eine Verbindung der Formel (IV),

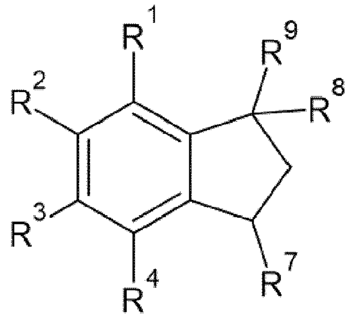

(IV),

in der zumindest einer der Reste $R^1$, $R^2$ oder $R^3$ für $SO_3X$ steht, und

in der die Reste $R^1$, $R^2$ und $R^3$, die nicht für $SO_3X$ stehen, und die Reste $R^7$, $R^8$ und $R^9$ jeweils unabhängig voneinander für Wasserstoff, COOX, SOsX, Amino, Nitro, Fluor, Chlor, Brom oder Alkyl stehen, und

in der $R^4$ für Amino steht,

und in der X für Wasserstoff, ein Alkalimetallion oder ein Erdalkalimetallion oder ein Ammoniumion steht.

Ebenfalls besonders bevorzugt ist die Sulfonsäure der Formel (I) eine Verbindung der Formel (V),

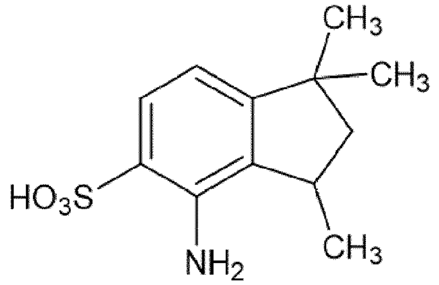

(V),

oder eine Verbindung der Formel (VI),

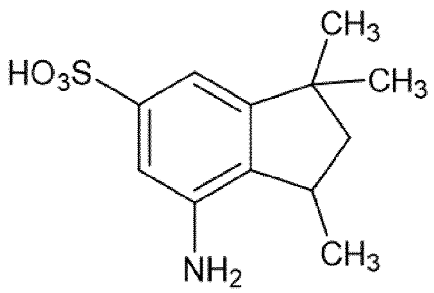

(VI),

oder deren Alkalimetallsalze, Erdalkalimetallsalze oder Ammoniumsalze.

Dabei steht Alkyl in den Formeln (I), (II), (III) oder (IV) bevorzugt für lineares oder verzweigtes $C_1$-$C_6$-Alkyl, das unsubstituiert, einfach oder mehrfach substituiert sein kann, oder dabei steht Alkyl in den Formeln (I), (II), (III) oder (IV) bevorzugt für $C_3$-$C_8$-Cycloalkyl steht, das unsubstituiert, einfach oder mehrfach substituiert sein kann, oder dabei steht Alkyl in den Formeln (I), (II), (III) oder (IV) bevorzugt für Aralkyl, das unsubstituiert, einfach oder mehrfach substituiert sein kann.

Besonders bevorzugt steht lineares oder verzweigtes $C_1$-$C_6$-Alkyl für Methyl, Ethyl, n-Propyl, Isopropyl, n-Butyl, sec-Butyl, Isobutyl, n-Pentyl, sec-Pentyl, 3-Pentyl, 2-Methylbutyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2,3-Dimethyl-2-butyl, 3,3-Dimethyl-2-butyl oder 3-Ethyl-1-butyl steht, wobei das lineare oder verzweigte $C_1$-$C_6$-Alkyl, mehrfach substituiert sein kann, beispielsweise 2-Methoxy-1-ethyl, 2-Ethoxy-1-ethyl, 3-Methoxy-1-propyl, 3-Ethoxy-1-propyl oder 1-Cyclopropylmethyl.

Ebenfalls besonders bevorzugt steht $C_3$-$C_8$-Cycloalkyl für Cyclopropyl, Cyclobutyl, Cyclopentyl, Cyclohexyl, Cycloheptyl, Cyclooctyl, 2-Methyl-cyclobutyl, 3-Methyl-cyclobutyl, 2-Methyl-cyclopentyl, 3-Methyl-cyclopentyl, 2-Methylcyclohexyl, 3-Methyl-cyclohexyl, 4-Methyl-cyclohexyl, 2-Methyl-cycloheptyl, 3-Methyl-cycloheptyl, 4-Methyl-cycloheptyl, 2-Ethyl-cyclobutyl, 3- Ethyl-cyclobutyl, 2-Ethyl-cyclopentyl, 3- Ethyl-cyclopentyl, 2- Ethyl-cyclohexyl, 3- Ethyl-cyclohexyl, 4- Ethyl-cyclohexyl, 2-Propyl-cyclobutyl, 3- Propyl-cyclobutyl, 2-Propyl-cyclopentyl, 3-Propyl-cyclopentyl, 2-Butyl-cyclobutyl, 3- Butyl-cyclobutyl, 2-Hydroxycyclopropyl oder 2-Fluorcyclopropyl steht, wobei das C3-C8-Cycloalkyl unsubstituiert, einfach oder mehrfach substituiert sein kann

Ebenfalls besonders bevorzugt kann Aralkyl für Benzyl, Phenethyl, 2-Furyl-methyl, 3-Furyl-methyl, 2-Pyridyl-methyl, 3-Pyridyl-methyl, 4-Pyridyl-methyl, 1-Naphthylmethyl oder 2-Naphthylmethyl steht, das unsubstituiert, einfach oder mehrfach substituiert sein kann.

Für alle bevorzugten und besonders bevorzugten Ausführungsformen wird das erfindungsgemäße Verfahren derart durchgeführt, wie es oben generell für Sulfonsäuren beschrieben wird.

Überraschenderweise kann mit dem erfindungsgemäßen Verfahren eine große Gruppe von aromatischen

Sulfonsäuren ökonomisch und effizient aus wässrigen Phasen abgetrennt werden.

**Beispiele**

In den Beispielen stehen die in Großbuchstaben geschriebenen Begriffe SÄURE, BASE und AMIN stellvertretend für die in der Tabelle 1 angegebenen spezifisch eingesetzten Stoffe. Die großgeschriebenen Begriffe PHASE A, PHASE B und PHASE C stehen stellvertretend für die in Tabelle 1 angeführten eingesetzten oder erhaltenen Phasen.

Variante A: Die wässrige Phase, die die zu entfernenden SÄURE und gegebenenfalls weitere Bestandteile enthält (Volumen der wässrigen Phase, Konzentration der SÄURE in der wässrigen PHASE A: siehe Tabelle 1), wurde mit BASE (Base und Menge der Base: siehe Tabelle 1) auf den gewünschten pH-Wert gestellt (pH-Wert: siehe Tabelle 1), und nachfolgend mit AMIN (Amin und Volumen des Amins: siehe Tabelle 1) überschichtet. Das daraus resultierende zweiphasige Gemisch wurde über einen Zeitraum von 15 Minuten bei Umgebungstemperatur intensiv durch Rühren oder Schütteln vermischt. Nach Beendigung der Vermischung bildeten sich zwei Phasen, wobei die obere PHASE B das AMIN und den Hauptanteil der SÄURE enthält und die untere PHASE C die wässrige Phase darstellt, die nur noch einen geringen Teil der SÄURE oder gar keine SÄURE und des AMINS enthält.

Variante B: Das AMIN (Amin und Volumen des Amins: siehe Tabelle 1) wurden vorgelegt, und die wässrigen Phase, die die zu entfernenden SÄURE und gegebenenfalls weitere Bestandteile enthält (SÄURE und deren Konzentration in der wässrigen PHASE A: siehe Tabelle 1), wurden zugegeben und das daraus resultierende Gemisch gegebenenfalls durch Rühren oder Schütteln intensiv vermischt. Dabei entsteht in der Regel ein einphasiges Gemisch. Anschließend wurde das Gemisch mit BASE (Base und Menge der Base: siehe Tabelle 1) auf den gewünschten pH-Wert eingestellt (pH-Wert: siehe Tabelle 1) und das daraus resultierende Gemisch über einen Zeitraum von 15 Minuten bei Umgebungstemperatur intensiv durch Rühren oder Schütteln vermischt. Nach Beendigung der Vermischung bildeten sich zwei Phasen, wobei die obere PHASE B das AMIN und den Hauptanteil der SÄURE enthält und die untere PHASE C die wässrige Phase darstellt, die nur noch einen geringen Teil der SÄURE und des AMINS enthält.

Variante C: Die wässrige Phase, die die zu entfernenden SÄURE und gegebenenfalls weitere Bestandteile enthält (Volumen der wässrigen Phase, Konzentration der SÄURE in der wässrigen PHASE A: siehe Tabelle 1), wurden vorgelegt, und AMIN (Amin und Volumen des Amins: siehe Tabelle 1) wurden zugegeben und das daraus resultierende Gemisch gegebenenfalls durch Rühren oder Schütteln vermischt. Dabei entsteht in der Regel ein einphasiges Gemisch. Anschließend wurde das Gemisch mit BASE (Base und Menge der Base: siehe Tabelle 1) auf den gewünschten pH-Wert eingestellt (pH-Wert: siehe Tabelle 1) und das daraus resultierende Gemisch über einen Zeitraum von 15 Minuten bei Umgebungstemperatur intensiv durch Rühren oder Schütteln vermischt. Nach Beendigung der Vermischung bildeten sich zwei Phasen, wobei die obere PHASE B das AMIN und den Hauptanteil der SÄURE enthält und die untere Phase die wässrige PHASE C darstellt, die nur noch einen geringen Teil der SÄURE und des AMINS enthält.

**Tabelle 1:** In Tabelle 1 sind die erfindungsgemäßen (Beispielnummern E1 bis E4) und nicht-erfindungsgemäßen (Beispielnummern N1 bis N2) Beispiele genauer mit deren Parametern angegeben.

| Beispielnummer | Variante | SÄURE | SÄURE in PHASE A | AMIN | BASE | Masse BASE | pH-Wert PHASE A | Konzentration SÄURE in PHASE B | Konzentration SÄURE in PHASE C |
|---|---|---|---|---|---|---|---|---|---|
| E1 | A | 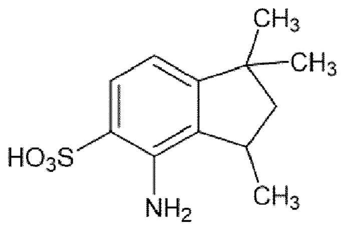 | 7,6 g in 100 ml wässriger Phase c(aq): 7,6 g/ 100 ml | 10 ml Cyclohexylamin | NaOH (Pastillen) | 3,0 g | 14 | 7,6 g in 10 ml c(org): 76 g/ 100 ml | <0,01 g in 100 ml c(aq): <0,01 g/ 100 ml |
| Extraktionszahl: >760 | | | | | | | | | |
| E2 | A | 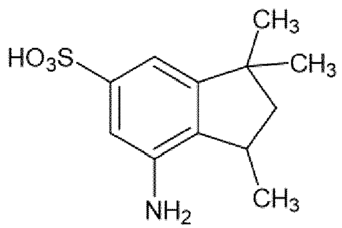 | 7,6 g in 100 ml wässriger Phase c(aq): 7,6 g/ 100 ml | 10 ml Cyclohexylamin | NaOH (Pastillen) | 3,0 g | 14 | 7,6 g in 10 ml c(org): 76 g/ 100 ml | <0,01 g / 100 ml c(aq): <0,01 g/ 100 ml |
| Extraktionszahl: >760 | | | | | | | | | |
| E3 | B | 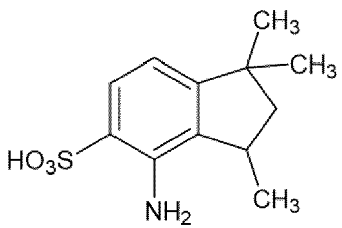 | 7,6 g in 100 ml wässriger Phase c(aq): 7,6 g/ 100 ml | 10 ml Cyclohexylamin | Natronlauge (32 Gew. %) | 9,5 g | 14 | 7,6 g in 10 ml c(org): 76 g / 100 ml | <0,01 g / 100 ml c(aq): <0,01 g / 100 ml |
| Extraktionszahl: >760 | | | | | | | | | |

(fortgesetzt)

| Beispielnummer | Variante | SÄURE | SÄURE in PHASE A | AMIN | BASE | Masse BASE | pH-Wert PHASE A | Konzentration SÄURE in PHASE B | Konzentration SÄURE in PHASE C |
|---|---|---|---|---|---|---|---|---|---|
| E4 | B | 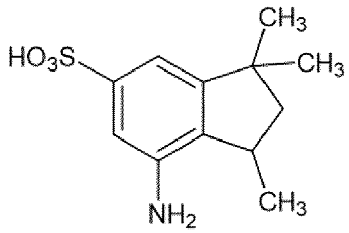 | 7,6 g in 100 ml wässriger Phase c(aq): 7,6 g/ 100 ml | 10 ml Cyclohexylamin | Natronlauge (32 Gew. %) | 9,5 g | 14 | 7,6 g in 10 ml c(org): 76 g / 100 ml | <0,01 g / 100 ml c(aq): <0,01 g / 100 ml |
| Extraktionszahl: >760 | | | | | | | | | |
| N1 (Nicht erfindungsgemäß) | A | 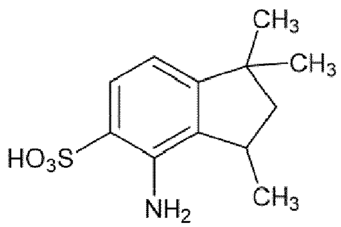 | 7,6 g in 150 ml wässriger Phase c(aq): 5,1 g/ 100 ml | 20 ml Tributylamin | NaOH (Pastillen) | 3,0 g | 14 | 0,76 g / 20 ml c(org): 3,8 g / 100 ml | 6,84 g / 150 ml c(aq): 4,56 g/ 100 ml |
| Extraktionszahl: 0,11 | | | | | | | | | |
| N2 (Nicht erfindungsgemäß) | A | 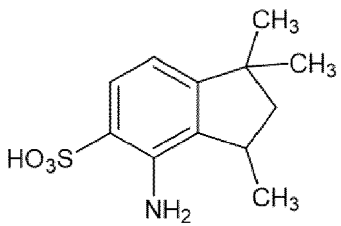 | 7,6 g in 150 ml wässriger Phase c(aq): 5,1 g/ 100 ml | 20 ml Triethylamin | NaOH (Pastillen) | 3,0 g | 14 | 0,65 g / 20 ml c(org): 3,25 g / 100 ml | 6,95 g / 150 ml c(aq): 4,63 g/ 100 ml |
| Extraktionszahl: 0,09 | | | | | | | | | |

**Patentansprüche**

1. Verfahren zur Abtrennung von Sulfonsäuren oder deren Salzen aus wässrigen Phasen, umfassend die Schritte

a) Einstellendes pH-Wertes der wässrigen Phase, in der sich die zumindest eine Sulfonsäure oder deren Salz befindet, auf pH 13 bis 15, und
b) Inkontaktbringen und Vermischung der wässrigen Phase aus Schritt a) mit zumindest einem Amin ausgewählt aus der Gruppe der alicylclischen und aromatischen Amine mit einer Anzahl von Kohlenstoffatomen im Molekül von 5 bis 12 und einer Anzahl von Stickstoffatomen im Molekül von 1 bis 2, wobei sich eine Mischung umfassend zumindest eine wässrige Phase und eine aminische Phase ausbildet, wobei die aminische Phase die zumindest eine abzutrennende Sulfonsäure oder deren Salz enthält, und
c) Trennung der wässrigen Phase und der aminischen Phase.

2. Verfahren nach Anspruch 1, in dem die Sulfonsäuren eine Verbindung der Formel (I),

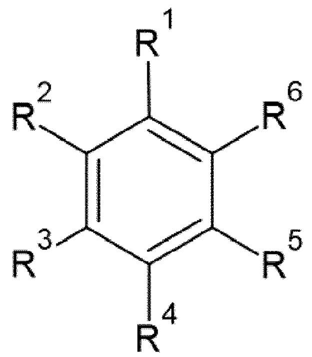

(I),

ist, in der zumindest einer der Reste $R^1$, $R^2$, $R^3$ oder $R^4$ für SOsX steht, und
in der die Reste $R^1$, $R^2$, $R^3$, und $R^4$, die nicht für SOsX stehen, jeweils unabhängig voneinander für Wasserstoff, Nitro, Fluor, Chlor, Brom oder Alkyl stehen und
in der $R^5$ und $R^6$ zusammen mit den Kohlenstoffatomen, mit denen sie verbunden sind, für einen anellierten alizyklischen 5-gliedrigen bis 7-gliedrigen Ring oder einen 6-gliedrigen aromatischen oder heteroaromatischen Ring bilden, der selbst ein oder mehrfach mit den Resten $R^7$, $R^8$, $R^9$ und $R^{10}$ substituiert sein kann, wobei $R^7$, $R^8$, $R^9$ und $R^{10}$ jeweils unabhängig voneinander für COOX, SOsX, Hydroxy, Amino, Nitro, Fluor, Chlor, Brom oder Alkyl stehen
und in der X für Wasserstoff, ein Alkalimetallion, ein Erdalkalimetallion oder ein Ammoniumion steht,

3. Verfahren nach Anspruch 1 oder 2, in die Verbindung der Formel (I) eine Verbindung der Formel (II),

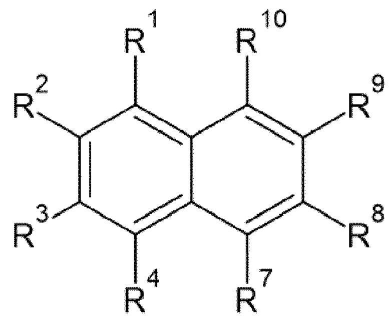

(II),

ist, in der die Reste $R^1$, $R^2$, $R^3$, $R^4$, $R^7$, $R^8$, $R^9$ und $R^{10}$ die für Formel (I) genannte Bedeutung haben.

4. Verfahren nach Anspruch 1 oder 2, in der die Verbindung der Formel (I) eine Verbindung der Formel (III),

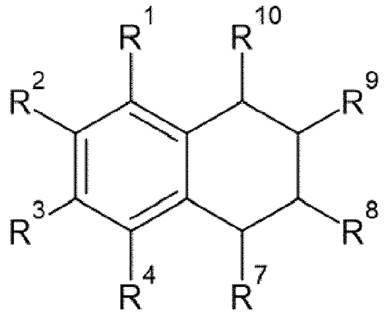

(III),

ist, in der die Reste $R^1$, $R^2$, $R^3$, $R^4$, $R^7$, $R^8$, $R^9$ und $R^{10}$ die für Formel (I) genannte Bedeutung haben.

**5.** Verfahren nach Anspruch 1 oder 2, in der die Verbindung der Formel (I) eine Verbindung der Formel (IV),

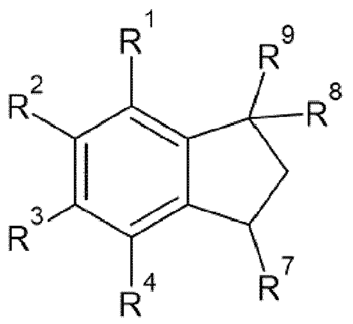

(IV),

ist,

in der zumindest einer der Reste $R^1$, $R^2$, $R^3$ oder $R^4$ für SOsX steht, und
in der die Reste $R^1$, $R^2$, $R^3$ oder $R^4$, die nicht für $SO_3X$ stehen, und die Reste $R^7$, $R^8$ und $R^9$ die für Formel (I) genannte Bedeutung haben.

**6.** Verfahren nach Anspruch 1 oder 2, in dem die Sulfonsäuren der Formel (I) eine Verbindung der Formel (IV),

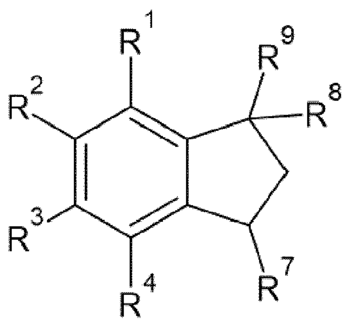

(IV),

ist,

in der zumindest einer der Reste $R^1$, $R^2$ oder $R^3$ für $SO_3X$ steht, und
in der die Reste $R^1$, $R^2$, $R^3$, die nicht für SOsX stehen, und die Reste $R^7$, $R^8$ und $R^9$ jeweils unabhängig voneinander für Wasserstoff, COOX, SOsX, Amino, Nitro, Fluor, Chlor, Brom oder Alkyl stehen, und
in der $R^4$ für Amino steht,
und in der X für Wasserstoff, ein Alkalimetallion oder ein Erdalkalimetallion oder ein Ammoniumion steht.

**7.** Verfahren nach Anspruch 1 oder 2, in dem die Sulfonsäuren der Formel (I) eine Verbindung der Formel (V),

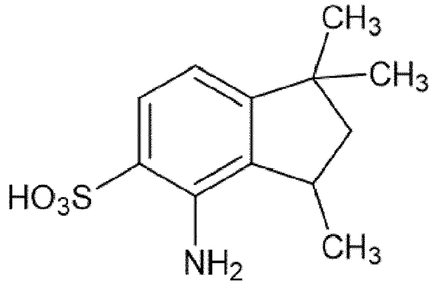

(V),

oder eine Verbindung der Formel (VI) darstellt

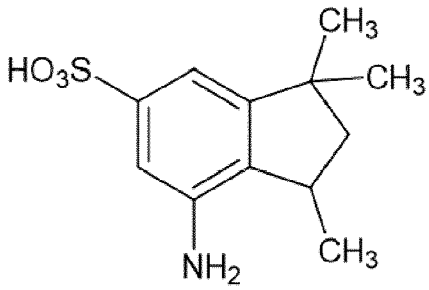

(VI),

oder deren Alkalimetallsalze, Erdalkalimetallsalze oder Ammoniumsalze.

**8.** Verfahren nach einem der Ansprüche 2 bis 6, in dem Alkyl in den Formeln (I), (II), (III) oder (IV) für lineares oder verzweigtes $C_1$-$C_6$-Alkyl steht, das unsubstituiert, einfach oder mehrfach substituiert sein kann, oder in der Alkyl für $C_3$-$C_8$-Cycloalkyl steht, das unsubstituiert, einfach oder mehrfach substituiert sein kann, oder in der Alkyl für Aralkyl steht, das unsubstituiert, einfach oder mehrfach substituiert sein kann.

**9.** Verfahren nach einem der Ansprüche 2 bis 6, in dem lineares oder verzweigtes $C_1$-$C_6$-Alkyl für Methyl, Ethyl, n-Propyl, Isopropyl, n-Butyl, sec-Butyl, Isobutyl, n-Pentyl, sec-Pentyl, 3-Pentyl, 2-Methylbutyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2,3-Dimethyl-2-butyl, 3,3-Dimethyl-2-butyl oder 3-Ethyl-1-butyl steht, wobei das lineare oder verzweigte $C_1$-$C_6$-Alkyl, mehrfach substituiert sein kann, beispielsweise 2-Methoxy-1-ethyl, 2-Ethoxy-1-ethyl, 3-Methoxy-1-propyl, 3-Ethoxy-1-propyl oder 1-Cyclopropylmethyl steht.

**10.** Verfahren nach einem der Ansprüche 2 bis 6, in dem $C_3$-$C_8$-Cycloalkyl für Cyclopropyl, Cyclobutyl, Cyclopentyl, Cyclohexyl, Cycloheptyl, Cyclooctyl, 2-Methyl-cyclobutyl, 3-Methyl-cyclobutyl, 2-Methyl-cyclopentyl, 3-Methyl-cyclopentyl, 2-Methyl-cyclohexyl, 3-Methyl-cyclohexyl, 4-Methyl-cyclohexyl, 2-Methyl-cycloheptyl, 3-Methyl-cycloheptyl, 4-Methyl-cycloheptyl, 2-Ethyl-cyclobutyl, 3- Ethyl-cyclobutyl, 2- Ethyl-cyclopentyl, 3- Ethyl-cyclopentyl, 2-Ethyl-cyclohexyl, 3- Ethyl-cyclohexyl, 4- Ethyl-cyclohexyl, 2-Propyl-cyclobutyl, 3- Propyl-cyclobutyl, 2-Propyl-cyclopentyl, 3-Propyl-cyclopentyl, 2-Butyl-cyclobutyl, 3- Butyl-cyclobutyl, 2-Hydroxycyclopropyl oder 2-Fluorcyclopropyl steht, wobei das C3-C8-Cycloalkyl unsubstituiert, einfach oder mehrfach substituiert sein kann.

**11.** Verfahren nach einem der Ansprüche 2 bis 6, in dem Aralkyl für Benzyl, Phenethyl, 2-Furyl-methyl, 3-Furyl-methyl, 2-Pyridyl-methyl, 3-Pyridyl-methyl, 4-Pyridyl-methyl, 1-Naphthylmethyl oder 2-Naphthylmethyl steht, das unsubstituiert, einfach oder mehrfach substituiert sein kann.

**12.** Verfahren nach einem der Ansprüche 1 bis 11, in dem in Schritt a) der pH der wässrigen Phase durch Base, ausgewählt aus der Gruppe Alkalimetallhydroxide und Erdalkalimetallhydroxide, bevorzugt aus Natriumhydroxid und Kaliumhydroxid, eingestellt wird.

**13.** Verfahren nach einem der Ansprüche 1 bis 12, in dem in Schritt b) das Amin ausgewählt aus der Gruppe Cyclohexylamin, Isophorondiamin und Anilin ist.

**14.** Verfahren nach einem der Ansprüche 1 bis 13, in dem die Schritte a), b) und/oder c) bei einer Temperatur von 0 bis 70 °C, bevorzugt von 15 bis 60 °C, besonders bevorzugt von 20 bis 50 °C durchgeführt werden.

**15.** Verfahren nach einem der Ansprüche 1 bis 14, in dem in Schritt b) das Massenverhältnis zwischen wässriger Phase und Amin von 1 : 1 bis 25 : 1, bevorzugt von 3 : 1 bis 15 : 1, beträgt.

Europäisches Patentamt
European Patent Office
Office européen des brevets

# EUROPÄISCHER RECHERCHENBERICHT

Nummer der Anmeldung
EP 23 21 9812

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 0 041 134 A2 (BASF AG [DE]) 9. Dezember 1981 (1981-12-09) * das ganze Dokument * ----- | 1-15 | INV. C07C303/44 C07C309/47 |
| A,D | US 3 719 703 A (FRITZ J ET AL) 6. März 1973 (1973-03-06) * das ganze Dokument * ----- | 1-15 | |

RECHERCHIERTE SACHGEBIETE (IPC)
C07C

Der vorliegende Recherchenbericht wurde für alle Patentansprüche erstellt

1

| Recherchenort | Abschlußdatum der Recherche | Prüfer |
|---|---|---|
| Den Haag | 3. Juni 2024 | Fritz, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 21 9812

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-06-2024

| Im Recherchenbericht angeführtes Patentdokument | Datum der Veröffentlichung | Mitglied(er) der Patentfamilie | Datum der Veröffentlichung |
|---|---|---|---|
| EP 0041134 A2 | 09-12-1981 | AT E7909 T1 | 15-06-1984 |
| | | DE 3020526 A1 | 10-12-1981 |
| | | EP 0041134 A2 | 09-12-1981 |
| | | JP S5724351 A | 08-02-1982 |
| US 3719703 A | 06-03-1973 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3719703 A
- EP 41134 A2


### In der Beschreibung aufgeführte Nicht-Patentliteratur


- **H. GAI et al.** *Journal of Cleaner Production*, 2018, vol. 201, 774-782